Europäisches Patentamt

European Patent Office

Office européen des brevets

(19)

(11) Publication number: 0 260 854
A1

(12) EUROPEAN PATENT APPLICATION

(21) Application number: 87307878.6

(22) Date of filing: 07.09.87

(51) Int. Cl.⁴: **A61K 9/22** , A61D 1/02

(30) Priority: 05.09.86 GB 8621484

(43) Date of publication of application:
23.03.88 Bulletin 88/12

(84) Designated Contracting States:
ES GR

(71) Applicant: NORBROOK LABORATORIES
LIMITED
Station Works
Newry, County Down BT35 6JP(GB)

(72) Inventor: Haughey, Edward
Station Works Camlough Road Newry BT35
6JP
Down Northern Ireland(GB)

(74) Representative: Wotherspoon, Graham et al
FITZPATRICKS 4 West Regent Street
Glasgow G2 1RS Scotland(GB)

(54) Intramammary infusion.

(57) An intramammary infusion comprises in a pharmaceutically acceptable vehicle a therapeutic or prophylactic dosage unit of a substance which is active against mammary infection and second, and optional subsequent, dosage units of active substance, particles of the said second and subsequent dosage units being microencapsulated within encapsulating membranes capable of degrading at preselected time intervals following administration.

EP 0 260 854 A1

## Intramammary Infusion

This invention relates to a veterinary intramammary infusion. More particularly, the invention relates to a therapeutic or prophylactic infusion for bovine mastitis, microbial mammary infection or similar problems which require intramammary introduction of medicaments.

A typical anti-mastitis dry cow (non-lactating) intramammary infusion would be a suspension in liquid paraffin of Cloxacillin Benzathine and Ampicillin Trihydrate. In general each infusion has effect over a period of around three weeks after administration. However, as the dry cow period is generally in excess of this, subsequent infusions are normally recommended. This is often inconvenient and costly. Furthermore if the cow should calve earlier than expected, shortly after infusion of the product, then the milk produced may continue to contain undesirable concentrations of the infusion for an unacceptable period. This, of course, may cause it to be deemed unfit for market and such milk would be subject to rejection, perhaps for some considerable time.

It is an object of this invention to obviate or mitigate the aforesaid disadvantages.

According to the present invention there is provided an intramammary infusion comprising in a pharmaceutically acceptable vehicle a therapeutic or prophylactic dosage unit of a substance which is active against mammary infection and second, and optional subsequent, dosage units of active substance, particles of the said second and subsequent dosage units being microencapsulated within encapsulating membrane capable of degrading at preselected time intervals following administration.

The microcapsules may be administered in a pharmaceutically acceptable vehicle in suspension capsules, mousse, liquid, paste, gel or the like. The active substance(s), together with any pharmaceutically acceptable additives, may be powdered or pelletted or similarly treated to facilitate delivery and microencapsulation.

A significant advantage obtained by utilising the infusion of the present invention is that it requires administration only once by a single infusion to obtain the full term of protection normally required. Additionally by containing the active substances within microcapsules of varying degradability, release of active substnaces may be controlled to provide a more uniform release profile. Most importantly the invention now provides a means of rapidly removing the drugs from the mammary system. In the event that a cow should calve and begin to lactate within the period of activity of the infusion, any residual active material may be removed within an acceptable period simply by milking the animal because the high milk yield after calving is adequate to achieve physically flushing out of the remaining intact microcapsules.

Preferably, the active substance is a mixture of Cloxacillin Benzathine and Ampicillin Trihydrate, the infusion being active for the treatment of prophylaxis of mastitis.

The encapsulating membrane may conveniently be selected from gelatin, an acrylate polymer, poly(DL-lactide-CO-glycolide) and poly-(hydroxybutyrate). These materials are all commercially available and the choice of material will be made by selection depending upon the known degradation/dissolution rate. The degree of degradation required may be varied in a manner known per se in the art for other purposes.

The particle size of the microencapsulated material is believed to be non-critical provided that it is small enough to introduce into the udder intramammarily and not so small that it may obstruct milk-producing structures within the udder. Thus suitable particle sizes lie in the range of from about 10 $\mu$m to 500 $\mu$m. Preferred particle sizes lie in the range of from about 25 $\mu$m to about 250 $\mu$m, more preferably from about 45 $\mu$m to about 150 $\mu$m.

An example of an infusion of this invention is based on a formulation in which the first dosage unit is :

Cloxacillin Benzathine    1-1000 mg.
Ampicillin Trihydrate    1-1000 mg.

The said first dosage unit is in the form of a micronised powder which is encapsulated and dispersed in a sterile liquid paraffin vehicle. Also dispersed in the vehicle are three subsequent dosage units in which powder particles, the said Cloxacillin Benzathine and Ampicillin Trihydrate are microencapsulated for delayed release after three, six and nine weeks thus giving a total of twelve weeks effect.

It will be understood that the invention has been described with reference to drugs known to have therapeutic effect against bovine mastitis but is not intended to be restricted thereto. Those skilled in the art will appreciate that the invention may be applied as preventative or curative measure for the delivery of drugs other than those exemplified above and for the purpose of combatting other diseases of the mammary system in dry or lactating mammalian livestock. Likewise the physical form of the drug prior to microencapsulation is a matter of choice and it may be selected according to the characteristics of the drug required.

## Claims

1. An intramammary infusion comprisisng, in a pharmaceutically acceptable vehicle, a therapeutic or prophylactic dosage unit of a substance which is active against mammary infection and second, and optional subsequent, dosage units of active substance, particles of the said second and subsequent dosage units being microencapsulated within encapsulating membranes capable of degrading at preselected time intervals following administration.

2. An intramammary infusion according to claim 1 wherein the microcapsules of active substance are in suspension in a pharmaceutically acceptable liquid vehicle.

3. An intramammary infusion according to claim 1 or claim 2 wherein the active substance is powdered prior to encapsulation.

4. An intramammary infusion according to any one of claims 1 to 3 wherein the active substance is a mixture of Cloxacillin Benzathine and Ampicillin Trihydrate, the infusion being active for the treatment of prophylaxis of mastitis.

5. An intramammary infusion according to any one of claims 1 to 4 wherein the particle size of the microencapsulated particles lies in the range of from 10 $\mu$m to 500 $\mu$m.

6. An intramammary infusion substantially as hereinbefore described.

7. A method of preparing an intramammary infusion comprising the steps of reducing at least one therapeutically-active substance to an administrable particle size, encapsulating the substance(s) in a degradable pharmaceutically acceptable encapsulation material to provide particles of therapeutically active substance(s) within encapsulation membranes of varying degradation or dissolution rate and dispersing same in a sterile pharmaceutically acceptable vehicle.

8. A method of preparing an intramammary infusion according to claim 8 wherein the therapeutically active substance(s) is (are) reduced to a micronised powder prior to encapsulation.

9. A method according to claim 9 wherein the microcapsules are dispersed in a sterile pharmaceutically acceptable liquid vehicle.

10. A method according to claim 9 or claim 10 wherein the active substance is a mixture of Cloxacillin Benzathine and Ampicillin Trihydrate, the infusion being active for the treatment or prophylaxis of mastitis.

11. A method according to any one of claims 7 to 10 wherein the particle size of the microencapsulated particles lies in the range of from 10 $\mu$m to 500 $\mu$m.

12. A method of treating a mammary disease in mammallian livestock comprising administering intramammarily an infusion comprising in a pharmaceutically acceptable vehicle a therapeutic or prophylactic dosage unit of a substance which is active against mammary infection and second, and optional subsequent, dosage units of active substance, particles of the said second and subsequent dosage units being microencapsulated within encapsulating membranes capable of degrading at preselected time intervals following administration.

European Patent Office

**PARTIAL EUROPEAN SEARCH REPORT**
which under Rule 45 of the European Patent Convention shall be considered, for the purposes of subsequent proceedings, as the European search report

Application number

EP 87 30 7878

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 076 068 (BEECHAM GROUP PLC) | | A 61 K 9/22 |
| | * Page 6, line 31 - page 7, line 15 * | 1,2 | A 61 D 1/02 |
| Y | | 3-11 | |
| | --- | | |
| Y | BIOLOGICAL ABSTRACTS, 1981, ref.no. 81 040635; Philadelphia, US K.B. SINGH et al.: "Clinical efficacy of orbenin LA and ampiclox LC in acute mastitis." & INDIAN J. VET. MED. 1984 (Publ. 1985), 4(2), 94-96. | | |
| | * Abstract * | 4,10 | |
| | --- | | |
| Y | EP-A-0 132 102 (SMITHKLINE BECKMAN) | | |
| | * Page 6, line 1 - page 7, line 24; figure 1 * | 3,5-11 | |
| | --- | | TECHNICAL FIELDS SEARCHED (Int. Cl.4) |
| A | US-A-4 011 312 (REUTER et al.) | | A 61 D |
| | * Column 2, lines 24-60 * | 5,11 | A 61 K |

**INCOMPLETE SEARCH** --- ./.

The Search Division considers that the present European patent application does not comply with the provisions of the European Patent Convention to such an extent that it is not possible to carry out a meaningful search into the state of the art on the basis of some of the claims.
Claims searched completely: 1-11
Claims searched incompletely:
Claims not searched: 12
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 23-11-1987 | EHRSAM |

## DOCUMENTS CONSIDERED TO BE RELEVANT

| Category | Citation of document with indication, where appropriate, of relevant passages | Relevant to claim |
|---|---|---|
| A | BIOLOGICAL ABSTRACTS, 1979, ref.no. 69075320; Philadelphia, US A.B. VILIM et al.: "Microbiological determination of penicillin G ampicillin and cloxacillin residues in milk." & J. ASSOC. OFF. ANAL. CHEM. (US), 1979, 62(6), 1247-50. * Abstract * | 4 |

CLASSIFICATION OF THE APPLICATION (Int. Cl.4)

TECHNICAL FIELDS SEARCHED (Int. Cl.4)